# EUROPEAN PATENT SPECIFICATION

(11) **EP 4 440 444 B1**
(45) Date of publication and mention of the grant of the patent: **07.10.2026**
(21) Application number: 22843576.4
(22) Date of filing: 30.11.2022
(51) Int. Cl.: A61B 10/02

(54) **MULTI-FACETED BIOPSY NEEDLE TIPS AND NEEDLES, NEEDLE SETS, AND DEVICES INCORPORATING THE SAME**
BIOPSIENADELSPITZEN UND -NADELN MIT MEHREREN FACETTEN, NADELSÄTZE UND VORRICHTUNGEN DAMIT
POINTES D'AIGUILLE DE BIOPSIE À FACETTES MULTIPLES ET AIGUILLES, ENSEMBLES D'AIGUILLES ET DISPOSITIFS INCORPORANT CELLES-CI

(30) Priority: 01.12.2021 US 202163284820 P
(43) Date of publication of application: 09.10.2024
(73) Proprietor: Hologic, Inc., Marlborough, MA 01752 (US)
(72) Inventor: ZHOU, Yi, Marlborough, Massachusetts 01752 (US); BLOZNALIS, Peter, Marlborough, Massachusetts 01752 (US)
(74) Representative: Carpmaels & Ransford LLP
(86) International application number: PCT/US2022/080633
(87) International publication number: WO 2023/102410

(56) References cited:
- US-A1- 2014 100 478
- US-A1- 2019 021 705

## Description

### Technical Field

The present disclosure generally relates to the field of soft tissue sampling and harvesting. More specifically, the disclosure relates to needle tip designs for biopsy needles, needle sets, and/or devices capable of sampling soft tissue.

### Introduction

The section headings used herein are for organizational purposes only and are not to be construed as limiting the subject matter described in any way.

In the practice of diagnostic medicine, it is often necessary or desirable to perform a biopsy, or to sample selected tissue from a living patient for medical evaluation. Cytological and histological studies of the biopsy sample can then be performed as an aid to the diagnosis and treatment of disease. Biopsies can be useful in diagnosing and treating various forms of cancer, including, for example, breast cancer, as well as other diseases in which a localized area of affected tissue can be identified.

Preoperative diagnosis with a percutaneous biopsy is generally preferred over surgical biopsy. With a percutaneous biopsy, a surgeon takes a tissue sample through the skin with a needle set, as opposed to making an actual surgical excision. One known needle set includes an elongate outer cannula (needle) having a pointed tissue piercing (penetrating) tip and a receiving aperture defined near its distal end (adjacent to the tissue piercing tip), and an inner cannula having an open distal end surrounded by an annular cutting blade. The inner cannula is slidably disposed within the outer cannula so that it can close the tissue receiving aperture, thereby cutting tissue prolapsing into the lumen of the outer cannula (needle) through the tissue receiving aperture.

Percutaneous image-guided needle biopsy, for example, has become the preferred biopsy method in the management of suspicious breast lesions detected by screening or during the assessment of clinical abnormalities. Percutaneous image-guided needle biopsy is recognized as a safe and cost-effective procedure, which allows for accurate diagnosis, decision-making, and when indicated treatment planning. Consequently, percutaneous image-guided breast biopsies have almost entirely replaced diagnostic surgical excisions, which were associated with longer hospital stays, higher costs, and possible complications, and have become the standard of care in the medical community.

There are also different technical options for breast tissue sampling, including, for example: fine-needle sampling (FNS), core needle biopsy (CNB), and vacuum-assisted biopsy (VAB), which may be used in conjunction with various imaging modalities for guidance. These different techniques are all available to diagnose palpable and nonpalpable breast lesions, with each generally be used for a different purpose. Accordingly, the different biopsy techniques adopt needles of different sizes and lengths. The diameter of a needle's lumen (opening) is described by gauge numbers, with smaller gauge numbers indicating larger needle diameters. Commonly applied needles, for example, have a lumen diameter ranging from about 0.4 mm (27 gauge) to about 4.6 mm (7 gauge).

VAB procedures generally require needle sets having a relatively small gauge ranging from about 12 to 7 gauge. Furthermore, the standard of care for breast biopsies has shifted to utilizing smaller gauge needles. Smaller gauge needles (with larger lumen diameters) are desirable, for example, to extract larger amounts of tissue and to collect multiple samples sequentially without having to remove the needle from the patient to provide enough tissue for the pathologists to make a diagnosis, thereby reducing the risk of false-negative results or pathological underestimation. Larger needles (having smaller gauges), however, also raise issues with patient comfort, as it is generally more difficult to penetrate a patient's skin with such needles.

It may, therefore, be desirable to provide needle tips for biopsy needles and/or needle sets, including, for example, small gauge biopsy needles and needle sets, having a design that improves the patient experience and facilitates patient comfort by more easily penetrating a patient's skin to collect soft tissue samples, while also enabling large scale manufacturing of the needles and/or needle sets for commercial use. It may be further desirable to provide needle tips for biopsy needles and/or needle sets that allow for decreased insertion forces as the needle enters the soft tissue, while simultaneously not increasing the length of the needle.

US 2019/021705 A1 describes a tissue penetrating tip, which includes a proximal base, and a sharp distal point aligned with the central longitudinal axis, wherein the tip has a plurality of concave surfaces extending from the proximal base to the sharp distal point wherein the adjacent surfaces intersect to form a plurality of concave curved cutting edges.

US 2014/100478 A1 describes needle tips comprising a plurality of concave faces, wherein each edge has a length that is greater or equal to the dimension of the outer perimeter of the canula.

### Summary

The present disclosure addresses one or more of the above-mentioned problems and/or achieves one or more of the above-mentioned desirable features. Other features and/or advantages may become apparent from the description which follows.

In particular, the present invention provides a biopsy needle as recited in claim 1. Optional features are recited in the dependent claims.

The present invention also provides a biopsy needle set for penetrating soft tissue as recited in claims 17-19 and 22.

The present invention also provides a biopsy device as recited in claims 20 and 21.

Additional objects and advantages will be set forth in part in the description which follows.

It is to be understood that both the foregoing general description and the following detailed description are exemplary and explanatory only and are not restrictive of the present disclosure. It should be understood that the present disclosure could be practiced without having one or more features of these exemplary aspects and embodiments. The present invention is defined by the claims.

### Brief Description of the Drawings

The accompanying drawings, which are incorporated in and constitute a part of this specification, illustrate some exemplary embodiments of the present disclosure and together with the description, serve to explain certain principles. These drawings depict only typical embodiments of the disclosed inventions and are not therefore to be considered limiting of its scope. In the drawings:
FIG. 1 is a partial, isometric view of an example embodiment of a needle having a distal tip portion formed in accordance with the present disclosure;
FIG. 2 is a partial, top view of the needle of FIG. 1;
FIG. 3 is a partial, side view of the needle of FIG. 1;
FIG. 4 is an enlarged view of the distal tip portion of the needle of FIG. 1;
FIG. 5 is a partial, side view of another example embodiment of a needle in accordance with the present disclosure;
FIG. 6 is a partial, side view of yet another example embodiment of a needle in accordance with the present disclosure;
FIG. 7 illustrates the relationship between an angle of a distal tip portion of a needle and the corresponding dead space of the needle;
FIG. 8 is a partial, bottom view of an alternative example embodiment of a distal tip portion of the needle of FIG. 1, in which the distal tip portion includes reliefs in accordance with the present disclosure;
FIG. 9 is a distal end view of the needle of FIG. 8;
FIG. 10 is a cross-sectional view of the needle of FIG. 8, taken through line A-A of FIG 9;
FIG. 11 is a perspective view of an embodiment of a biopsy device incorporating an example embodiment of a needle set including the needle of FIG. 1; and
FIGS. 12 and 13 are partial, perspective views of the needle set of FIG. 11.

### Detailed Description of the Illustrated Embodiments

As described above, small gauge needles, having larger lumen diameters, have become the standard of care for breast biopsies. Smaller gauge needles, for example, may extract larger amounts of tissue and collect multiple samples sequentially without having to remove the needle from the patient. Such biopsy techniques may, therefore, improve the patient experience (i.e., by reducing the number of needle sticks required during the biopsy), while also reducing the risk of false-negative results or pathological underestimation (i.e., since more tissue may be collected during the biopsy procedure). It is, however, generally more difficult to penetrate a patient's skin with such needles, as the penetration force required to insert the needle also increases with the size (i.e., diameter) of the needle. Accordingly, to improve the functionality of a biopsy device and to improve the patient experience, it is desirable that biopsy needle designs and, in particular, small gauge biopsy needle designs, minimize the forces required to insert the needle into a patient's soft tissue (i.e., are designed to have relatively small insertion forces into soft tissue). As sharper needles generally have smaller insertion forces, exemplary embodiments of the present disclosure contemplate a needle tip design that results in a sharp needle, and which may be used with both small and large gauge needles.

Embodiments of the present invention provide a distal needle tip portion having a multi-faceted tissue piercing element with three distal planar cutting edges converging to form a sharp, distal point and three proximal planar cutting edges extending proximally from a respective one of the three distal planar cutting edges. Each of the proximal cutting edges forms a first angle with respect to a central longitudinal axis of the tissue piercing element and each distal cutting edge forms a second angle relative to the central longitudinal axis of the tissue piercing element, wherein the second angle is smaller than the first angle. In this manner, biopsy needles having a distal tip portion formed in accordance with the present invention have a multi-faceted tissue piercing (penetrating) tip, such that a cross-section of the biopsy needle is minimized at the distal tip. In accordance with the present invention, the distal tip portion described herein is used in biopsy needles, and needle sets, each having a distal tissue piercing (penetrating) tip including a first transitional facet and a second piercing facet, wherein an angle of a transitional facet (a first angle) is greater than an angle of a piercing facet (a second angle). Further, the distal tip includes a plurality (or set) of transitional facets and a plurality (or set) of piercing facets, wherein the plurality of transitional facets are positioned immediately adjacent to the plurality of piercing facets, with the piercing facets forming the distal end point of the distal tip of the biopsy needle and being configured to pierce (penetrate) the tissue of the patient's skin. The distal needle tip described herein can be used on biopsy needles of any size (i.e., any gauge) and any configuration (e.g., can be used as the penetrating tip on any stylet, inner cannula, outer cannula, or trocar) that is used to penetrate or pierce soft tissue including, but not limited to, breast tissue.

In one example embodiment, the contemplated multi-faceted needle tips may be used in conjunction with needle sets including an elongate outer cannula or trocar that functions as a needle, and an inner cannula that is slidably disposed within the outer cannula. As described above, the elongate outer cannula has, at the distal end, a tissue piercing tip and a tissue receiving aperture, and the inner cannula has an open distal end surrounded by an annular cutting blade. In this manner, a physician may collect a tissue sample by: (1) piercing a patient's skin with the tissue piercing tip of the outer cannula, (2) positioning the receiving aperture of the outer cannula such that tissue for collection prolapses into the aperture, and (3) sliding the inner cannula within the outer cannula to close the tissue receiving aperture, thereby cutting the prolapsing tissue with the annular cutting blade of the inner cannula. Various known biopsy devices, such as those described in U.S. Patent No. 9,456,808, entitled "Biopsy Device with Automatic Biopsy Parameter Adjustment" and issued October 4, 2016; U.S. Patent No. 10,022,110, entitled "Biopsy Device" and issued on July 17, 2018; and U.S. Patent No. 8,808,200, entitled "Surgical Device and Method of Using Same" and issued on August 19, 2014, may utilize such needle sets.

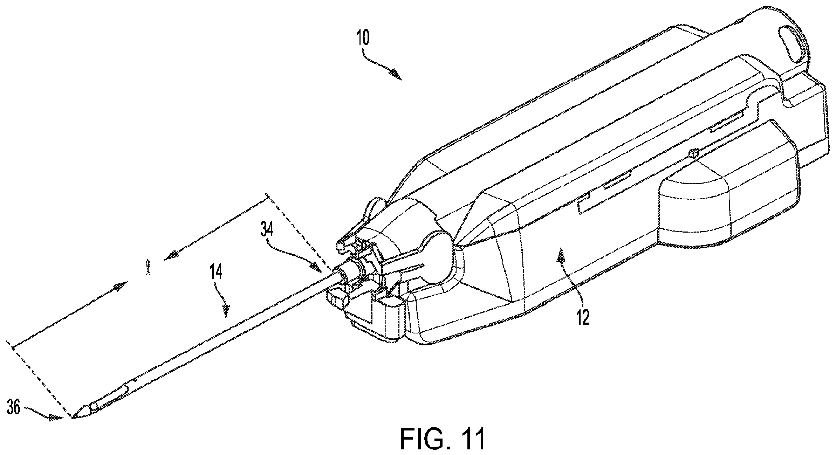

FIG. 11 illustrates a biopsy device 10 in accordance with one embodiment of the present disclosure. The biopsy device 10 includes a reusable body portion 12 and a disposable needle set 14. The body portion includes components configured to perform a tissue biopsy using the needle set 14. These components may include, for example, a drive assembly configured to drive movement of the components of the needle set 14. An exemplary drive system is described in U.S. Patent No. 10,022,110, entitled "Biopsy Device" and issued on July 17, 2018, and U.S. Patent No. 11,045,172, entitled "Biopsy Device" and dated June 29, 2021. The drive assembly can include one or more motors known in the art, including, for example, electrical, pneumatic, or hydraulic motors. The body portion 12 may also include a controller (e.g., a computer processor) configured to control the motors in the drive assembly and thereby control movement of the components of the needle set 14.

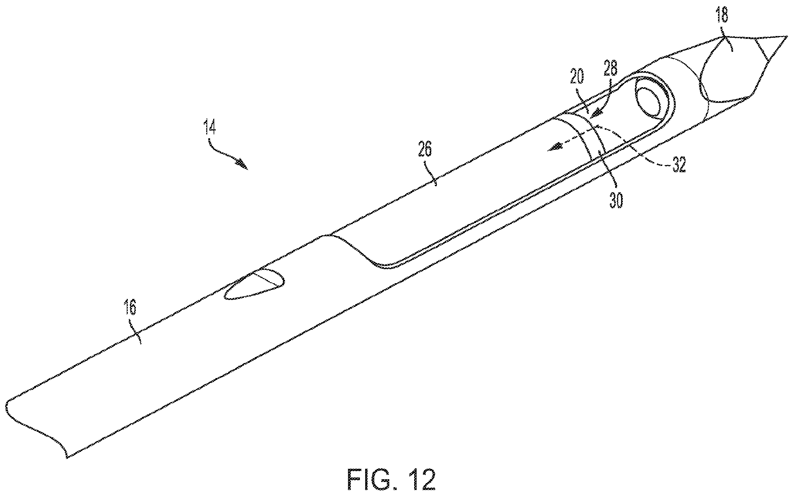

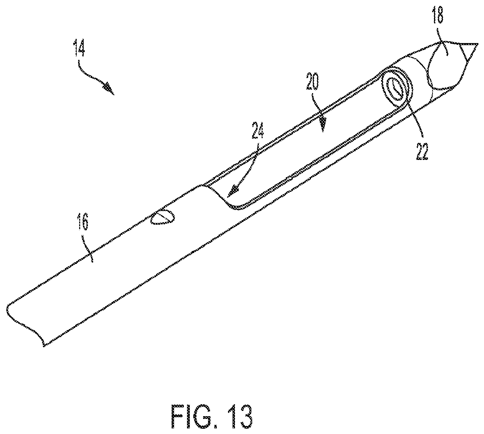

FIGS. 12 and 13 illustrate respective distal portions of the needle set 14. The needle set 14 includes an outer cannula or trocar 16 having a distal tissue piercing tip 18 formed in accordance with the present teachings and an inner cannula 26 having an open distal end 28 that is surrounded by an annular cutting blade 30. The outer cannula 16 defines an outer cannula lumen 24 and a tissue receiving aperture 20, adjacent to the distal tissue piercing tip 18, in communication with the outer cannula lumen 24. The inner cannula 26 is slidably disposed within the outer cannula lumen 24, such that when the inner cannula 26 is in its distal-most position in the outer cannula lumen 24, the inner cannula 26 closes the tissue receiving aperture 20 in the outer cannula 16. In this manner, during a biopsy procedure, the inner cannula 26 is configured to cut any tissue that extends within the receiving aperture 20 via the annular cutting blade 30 and collect the cut tissue into its open distal end 28, as the inner cannula 26 closes the tissue receiving aperture 20 of the outer cannula 16.

As shown in FIG. 13, in which the inner cannula 26 is not shown for clarity, in various exemplary embodiments, a cutting board 22 may also be disposed in the outer cannula lumen 24 in a position distal to the tissue receiving aperture 20. The cutting board 22 is configured to seal the open distal end 28 of the inner cannula 26 when the inner cannula 26 is in contact with the cutting board 22. This seal prevents fluids introduced into the outer cannula lumen 24 from being aspirated through the open distal end 28 and the inner cannula lumen 32 and bypassing the biopsy site. Instead, the fluids are delivered to the biopsy site through the outer cannula lumen 24 and the tissue receiving aperture 20.

Other aspects of the exemplary biopsy devices are described in U.S. Patent No. 9,585,639, entitled "Biopsy Device Arming Mechanism" and dated March 7, 2017; U.S.

Patent No. 10,022,110; U.S. Patent No. 9,456,808; U.S. Patent No. 8,808,200; and U.S.

Patent No. 9,844,363, entitled "Biopsy Device with Aspiration Valve" and dated December 19, 2017.

Those of ordinary skill in the art will understand that the biopsy device 10, including body portion 12 and needle set 14 illustrated in FIGS. 11-13 and described above are exemplary only, and that the multi-faceted trocars, needle tips, and needles of the present disclosure may be used in conjunction with various types and configurations of surgical instruments, biopsy devices, body portions/sleeves, stylets, needles, and needle sets, used for various types of applications, including but not limited to breast biopsies, and in various settings, without departing from the scope of the present disclosure. Those of ordinary skill in the art will also understand that the disclosed needle tips (e.g., the distal tissue piercing tips) may form the distal end of an outer cannula of a needle set and/or the distal end of an inner cannula of a needle set. Furthermore, those of ordinary skill in the art will understand that the disclosed needles (e.g., cutting elements) and needle sets (e.g., needle set 14), including an outer cannula or trocar (e.g. outer cannula 16) and an inner cannula (e.g., inner cannula 26) may be made from various high strength materials, including, but not limited to surgical grade stainless steel, MP35N (nonmagnetic, nickel cobalt alloy), other cobalt-chromium alloys, NiTi alloys, ceramics, glasses, high strength polymeric materials, and/or combinations thereof.

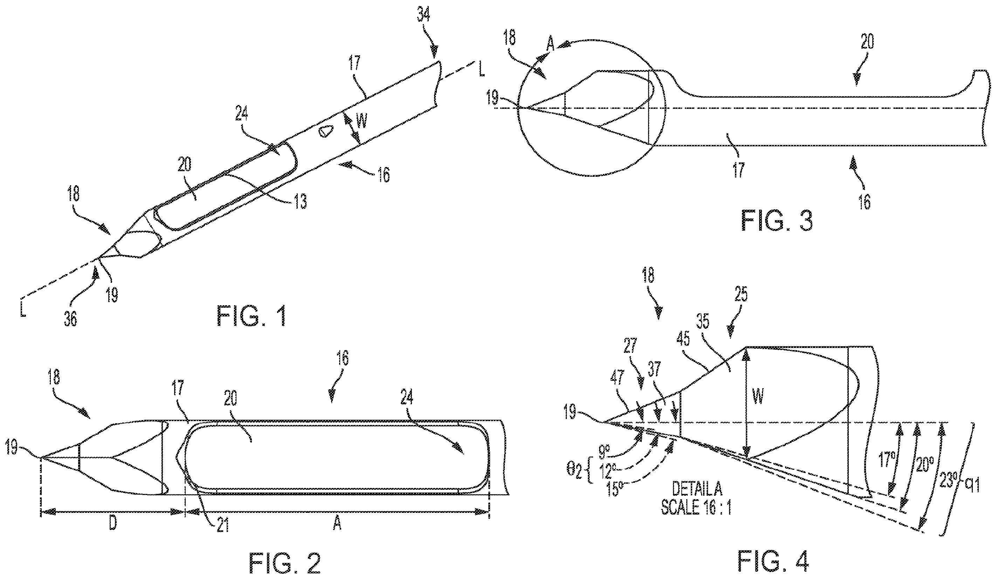

In accordance with standard practice, as will be understood by those of ordinary skill in the art, embodiments of the present disclosure contemplate that the needle tips (e.g., trocars, stylets, cannulas) of the present disclosure may be used in conjunction with various needles having various sizes and shapes (i.e., gauges and cross-sectional shapes), including, for example, needles having dimensions (e.g., widths and heights) corresponding to a 7-gauge needle through a 12-gauge needle. With reference to the embodiment of FIGS. 11-13, for example, the outer cannula 16 of the needle set 14 may have a relatively small gauge for biopsy procedures. In one example embodiment of the present disclosure, the outer cannula 16 is a 7-gauge to an 8-gauge needle, with a lumen having a width w (see FIG. 1), for example, a diameter, ranging from about 4.5 mm to about 5.6 mm. It should be noted that the range in the diameter (width) may incorporate variations between inner and outer diameters of the needle as well as variations in the cross-sectional shape of the needle. In another example embodiment, the outer cannula 16 is a 9-gauge to a 10-gauge needle, with a lumen having a width w ranging from about 3.3 mm to about 4 mm. In yet another example embodiment, the outer cannula 16 is a 11-gauge to a 12-gauge needle, with a lumen having a width w ranging from about 2.6 mm to about 3.0 mm. In various example embodiments, the cross-sectional shape of the needle may vary, for example, it may be circular or oval. Those of ordinary skill in the art will further understand, however, that the needles (trocars, stylets, cannulas) and needle sets in accordance with the present disclosure may have various gauges with various corresponding dimensions, based for example on the shape and manufacturer of the needle, to be used in conjunction with various types and configurations of surgical instruments and biopsy devices, for various types of applications, without departing from the scope of the present disclosure and claims.

FIGS. 1-4 illustrate one embodiment of a 7-gauge outer cannula or trocar 16 in accordance with the present disclosure. The outer cannula 16 includes an elongate body 17 and a tissue piercing tip 18. The elongate body 17 extends between a proximal end 34 and a distal end 36 of the outer cannula 16, such that the elongate body 17 defines a central longitudinal axis L of the cannula 16 (see FIGS. 1 and 11). The elongate body, for example, includes a sidewall 13 defining a lumen that extends along the central longitudinal axis L, with the tissue piercing tip 18 defining a distal end point 19 of the elongate body 17. Various embodiments contemplate an outer cannula having a length *ℓ* (see FIG. 11), extending along the central longitudinal axis, ranging from about 8 mm to about 16 mm. The length *ℓ* of a needle (i.e., outer cannula) may vary, however, depending on the needle's compatibility with a particular manufacturer and the sampling location (i.e., the location of a patient's lesion), as would be understood by those of ordinary skill in the art. As illustrated, for example, in FIG. 11 with reference to the proximal and distal ends 34 and 36 of the outer cannula or trocar 16, the terms "distal" and "proximal" refer to the orientation of a component relative to a device in which the outer cannula or trocar 16 is connected. In other words, with reference to FIG. 11, proximal signifies that a recited component is oriented closer to the body portion 12 than another component and distal signifies that a recited component is oriented further away from the body portion 12 than another component.

As best illustrated perhaps in the enlarged view of FIG. 4, to decrease the insertion forces of the outer cannula 16 (needle), the tissue piercing tip 18 of the outer cannula 16 is a multi-faceted tip, such that the distal tip 18 includes: 1) a first set of facets 25 defined by three first faces 35 and three first cutting edges 45, where adjacent faces 35 are connected to one another by cutting edges 45, and 2) a second set of facets 27 distal to the first set of facets 25, which are defined by three second faces 37 and three second cutting edges 47, where adjacent faces 37 are connected to one another by cutting edges 47. In this manner, each of the first cutting edges 45 is formed by adjacent faces 35 of the first three faces 35, and each of the second cutting edges 47 is formed by adjacent faces 37 of the second three faces 37. As illustrated in FIG. 4, the three first faces 35 and the three second faces 37 are all planar surfaces, such that the first cutting edges 45 and the second cutting edges 47 are all straight (planar) cutting edges, as described further below. In various additional embodiments, the three first faces 35 and the three first cutting edges 45 are all symmetrical to each other, and the three second faces 37 and the three second cutting edges 47 are all symmetrical to each other. In various additional embodiments, the three first faces 35 and the three first cutting edges 45 are all not all symmetrical to each other, and the three second faces 37 and the three second cutting edges 47 are not all symmetrical to each other, such that the needle tip has different sized faces on either side of a cutting edges.

As further illustrated in FIG. 4, each of the first cutting edges 45 is a straight (planar) edge that forms a first angle θ₁ relative to the central longitudinal axis L (thereby creating first faces 35 each having a first tapered surface) and each of the second cutting edges 47 is a straight (planar) edge that forms a second angle θ₂ relative to the central longitudinal axis L (thereby creating second faces 37 each having a second tapered surface). The first angle θ₁ is greater than the second angle θ₂, such that the three second cutting edges 47 converge at a sharper point (i.e., at the distal end point 19 of the tissue piercing tip 18) then the first cutting edges 45 would have converged (i.e., if the tip 18 did not include the second set of facets). In this manner, a cross-section of the outer cannula 16 is minimized at the tissue piercing tip 18. In other words, the tissue piercing tip 18 is designed to include a first set of transitional facets 25 and a second set of piercing facets 27, wherein an angle θ₁ of the transitional facets 25 is greater than an angle θ₂ of the piercing facets 35, such that the piercing facets 27 form a pyramidal tip that extends distally from the transitional facets 25. As illustrated in the embodiment of FIGS. 1-4, which shows an outer cannula 16 having a first angle θ₁ of about 20 degrees and a second angle θ₂ of about 12 degrees, each first cutting edge 45 forms a first angle θ₁ ranging between about 17 degrees and 23 degrees relative to the central longitudinal axis L (the full range of the first angle θ₁ being shown in dash for illustrative purposes), and each second cutting edge 47 forms a second angle θ₂ ranging between about 9 degrees and about 15 degrees relative to the central longitudinal axis L (the full range of the second angle θ₂ being shown in dash for illustrative purposes).

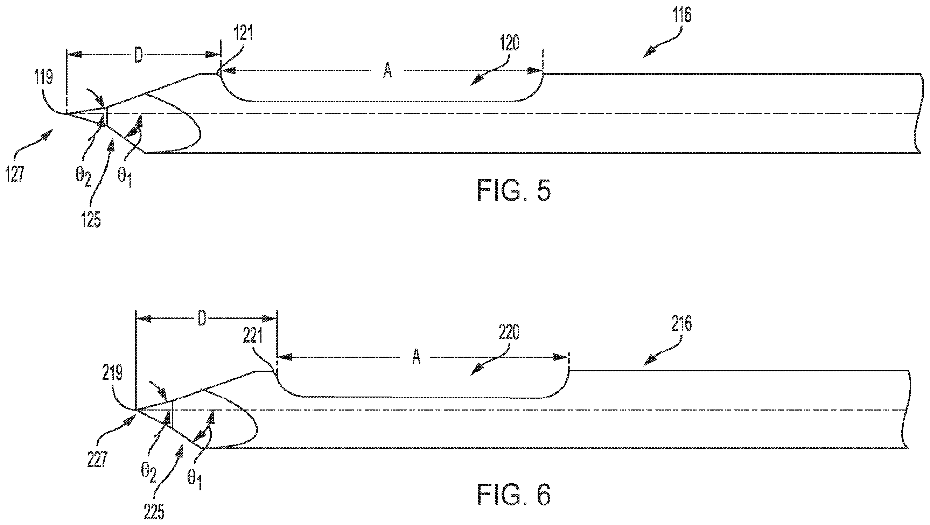

Those of ordinary skill in the art will understand that the outer cannula 16 illustrated in FIGS. 1-4 and described above is exemplary only, and that the outer cannulas (trocars or needles) in accordance with the present disclosure may have various designs and configurations, including various combinations of first sets of transitional facets and second sets of piercing facets, tapering at various combinations of first angles θ₁ and second angles θ₂, without departing from the present disclosure and claims. FIGS. 5 and 6 contemplate and illustrate, for example, several alternative embodiments of the present disclosure, with FIG. 5 illustrating a multi-faceted outer cannula 116 having a smaller second angle θ₂ (resulting in steeper pyramidal tip and a sharper distal end point 119) in comparison to cannula 16 of FIGS. 1-4 and FIG. 6 illustrating a multi-faceted outer cannula 216 having a larger second angle θ₂ (resulting in a blunter distal end point 219) in comparison to the cannula 16 of FIGS. 1-4. The outer cannula 116 has a first set of transitional facets 125, each tapering at a first angle θ₁ of about 20 degrees, and a second set of piercing facets 127, each tapering at a second angle θ₂ of about 9 degrees. While the outer cannula 216 has a first set of transitional facets 225, each tapering at a first angle θ₁ of about 20 degrees, and a second set of piercing facets 227, each tapering at a second angle θ₂ of about 15 degrees.

As discussed above, it will also be understood by those of ordinary skill in the art that the needle tips contemplated by the present disclosure may be used in conjunction with needles having various gauges, including but not limited to the 7-gauge needles discussed above, and, for example, also contemplate using such trocars with 8 through 12-gauge needles. In one example embodiment of the present disclosure, the outer cannula is a 7-gauge to an 8-gauge needle, with piercing faucets having a width W (see FIG. 4) ranging from about 4.4 mm to about 4.8 mm and having a first angle θ₁ ranging from about 17 degrees to about 23 degrees and a second angle θ₂ of about 9 degrees to about 15 degrees. In another embodiment, the outer cannula is a 9-gauge to a 10-gauge needle, with piercing faucets having a width W ranging from about 3.1 mm to about 3.5 mm and having a first angle θ₁ ranging from about 16 degrees to about 22 degrees and a second angle θ₂ of about 8 degrees to about 14 degrees. In yet another embodiment, the outer cannula is a 11-gauge to a 12-gauge needle, with piercing faucets having a width W ranging from about 2.2 mm to about 2.6 mm and having a first angle θ₁ ranging from about 13 degrees to about 15 degrees and a second angle θ₂ of about 8 degrees to about 10 degrees.

Those of ordinary skill in the art will also understand that the tissue piercing tip of the outer cannula or trocar may be made from various materials, including, for example, surgical stainless steel (e.g., 17-4 stainless steel). And, that the transitional and piercing facets may be formed on the tissue piercing tip using various known manufacturing methods and/or techniques, including, for example, by being ground into the tissue piercing tip via, for example, a grinding wheel. In various embodiments, the faces of the facets may then be electro-polished to increase the sharpness of the cutting edges.

As discussed above, although using smaller gauge needles (needles with larger widths/diameters) offer certain benefits, it is generally more difficult to penetrate a patient's skin with such needles, as the penetration force required to insert the needle also increases with the size (i.e., diameter) of the needle. Accordingly, it is desirable that the first and second angles θ₁ and θ₂ of the tissue piercing tip be relatively small to minimize the forces required to insert the needle into a patient's tissue. Angle selection, however, also involves other considerations and factors, including, for example, a dead space D that is created by the tissue piercing tip and the fragility of the tissue piercing tip. Although not wanting to be bound by any theories, the inventors found that: (1) the range discussed above for the first angle θ₁ allows the first set of transitional facets to penetrate tissue, while not excessively increasing the dead space D of the outer cannula. In other words, the disclosed range creates a transitional portion of the distal tip of the outer cannula that results in an acceptable dead space D (as will be understood by those of ordinary skill in the art and explained further below), without requiring a user to exert excessive penetration forces to insert the outer cannula, and (2) the above discussed range for the second angle θ₂ optimizes the second set of piercing facets ability to penetrate tissue, while not making the distal end point of the outer cannula too fragile.

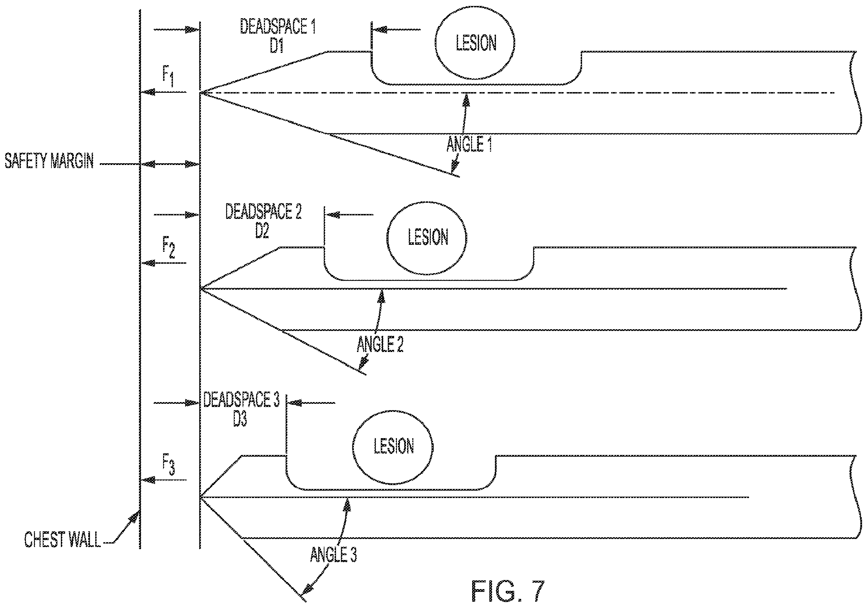

As used herein, the term "dead space" refers to a distance D between the distal end point 19, 119, 219 of the outer cannula 16, 116, 216 and a distal edge 21, 121, 221 of a tissue receiving aperture 20, 120, 220 (see FIGS. 2, 5, and 6). As illustrated in FIG. 7, when, for example, taking a biopsy of a suspected breast lesion, to allow access to tissue close to a chest wall (i.e., closer to a surface of the skin), it is advantageous to minimize the dead space D. In other words, a smaller dead space D allows access to tissue that is closer to the chest wall. It was additionally found that the dead space D is inversely related to the size of the first angle θ₁ and is therefore also inversely related to the amount of insertion force required to penetrate the skin (e.g., chest wall). Accordingly, with reference to FIG. 7: (1) a relatively small angle (angle 1) will result in a relatively small insertion force F₁ and a relatively large dead space D₁, (2) a relatively medium angle (angle 2) will result in a relatively medium insertion force F₂ and a relatively medium dead space D₂, and (3) a relatively large angle (angle 3) will result in a relatively large insertion force F₃ and a relatively large dead space D₃.

The example outer cannulas of FIGS. 1-6, which for exemplary purposes each have a first set of transitional facets 25, 125, 225 tapering at a first angle θ₁ of about 20 degrees, may each result in a dead space D of about .33 inches (8.4 mm) and have an aperture length A of about .79 inches (20 mm). Based on the present disclosure, a given application, and a sample location (e.g., legion proximity to the chest wall), one of ordinary skill in the art will be able to choose a second angle θ₂ to achieve an acceptable dead space D. For example, in embodiments of the present disclosure in which the outer cannula is a 7-gauge to an 8-gauge needle, an acceptable minimum dead space D may be about 9.5 mm to about 11 mm. And, in embodiments in which the outer cannula is a 9-gauge to a 12-gauge needle, an acceptable minimum dead space D may be about 8 mm to about 9 mm.

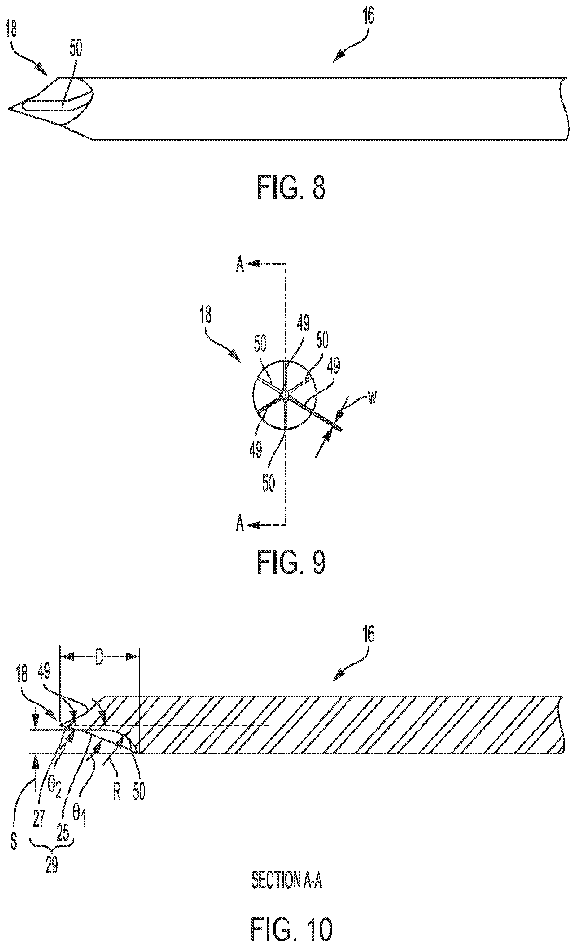

As discussed above, those of ordinary skill in the art will understand that the embodiments illustrated and described with relation to FIGS. 1-6 and 11-13 are exemplary only and that the disclosed biopsy devices, needle tips, needle sets, stylets, and inner and outer cannulas may be modified and/or include additional features without departing from the scope of the present disclosure and claims. It has also been found, for example, that the insertion force of a needle is related to the cross-sectional area of the needle tip (e.g., the tissue piercing tip 18). Accordingly, various additional embodiments of the present disclosure contemplate, for example, outer cannulas with multi-faceted tissue piercing tips (i.e., needle tips) including reliefs. As illustrated in FIGS. 8-10, in one example embodiment, the tissue piercing tip 18 of the outer cannula 16 may include one or more reliefs 50 extending along the central longitudinal axis L (three reliefs 50 are shown in the embodiment of FIGS. 8-10 (see FIG. 9)). In one embodiment, each of the reliefs 50 extends along a facet surface 29 of the tip 18 that is formed by a respective pair of transitional and piercing facets 25 and 27. As discussed above, each surface 29 is created by two different angle planes (e.g., each transitional facet tapers at a first angle θ₁ and each piercing facet tapers at a second angle θ₂), with each relief 50 creating a concave surface extending along the facet surface 29 between the planar cutting edges 45 and 47. In this manner, each relief 50 functions to remove material from the faces of the tissue piercing tip 18 to reduce the cross-sectional area of the tissue piercing tip 18, and thereby reduce the force required for insertion of the tissue piercing tip 18.

As shown best in the cross-sectional view of FIG. 10, in various embodiments, each relief 50 may have a radius of curvature R, that results in a relief 50 with a lateral span S (with relation to the central longitudinal axis L). As shown in the top view of FIG. 9, in this embodiment, removal of the three reliefs 50 forms three planar cutting edge surfaces 49 (each formed by a respective pair of cutting edges 45 and 47) each having a width w of about .01 inches (.25 mm). Taking into account the disclosed relationship of the needle tip's cross-sectional area to its insertion force, one of ordinary skill in the art will be able to choose appropriate values for the radius of curvature R, lateral span S, and cutting edge width w, for a particular application. For example, smaller values of S (e.g., as small as .03 inches (0.762mm)), along with correspondingly larger values of R (e.g., up to 1.0 inches (25.4mm)) and w (e.g., up to .05 inches (1.27mm)), will result in less cross-sectional area reduction and correspondingly less insertion force reduction. In embodiments of the present disclosure in which the outer cannula is a 7-gauge to an 8-gauge needle, for example, the radius of curvature R may range from about 3.5 mm to about 3.7 mm and the lateral span S may range from about 8.1 mm to about 8.3 mm. In embodiments in which the outer cannula is a 9-gauge to an 10-gauge needle, the radius of curvature R may range from about 2.5 mm to about 2.7 mm and the lateral span S may range from about 6.4 mm to about 6.6 mm And, in embodiments in which the outer cannula is a 11-gauge to a 12-gauge needle, the radius of curvature R may range from about 1.9 mm to about 2.1 mm and the lateral span S may range from about 6.4 mm to about 6.6 mm. Those of ordinary skill in the art will understand, however, that the multi-faceted tissue piercing tips contemplated by the present disclosure may include reliefs with various configurations, positions, and/or dimensions without departing from the scope of the present disclosure and claims.

This disclosure described some examples of the present technology with reference to the accompanying drawings, in which some of the possible examples were shown. Other aspects can, however, be embodied in many different forms and should not be construed as limited to the examples set forth herein. Rather, these examples were provided so that this disclosure was thorough and complete and fully conveyed the scope of the possible examples to those skilled in the art.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

This description and the accompanying drawings that illustrate exemplary embodiments should not be taken as limiting. Various mechanical, compositional, structural, electrical, and operational changes may be made without departing from the scope of the invention as defined by the claims. In some instances, well-known structures and techniques have not been shown or described in detail so as not to obscure the disclosure. Furthermore, elements and their associated features that are described in detail with reference to one embodiment may, whenever practical, be included in other embodiments in which they are not specifically shown or described. For example, if an element is described in detail with reference to one embodiment and is not described with reference to a second embodiment, the element may nevertheless be included in the second embodiment.

It is noted that, as used herein, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

Further, this description's terminology is not intended to limit the disclosure. For example, spatially relative terms-such as "beneath," "below," "lower," "above," "upper," "forward," "front," "behind," and the like-may be used to describe one element's or feature's relationship to another element or feature as illustrated in the orientation of the figures. These spatially relative terms are intended to encompass different positions and orientations of a device in use or operation in addition to the position and orientation shown in the figures. For example, if a device in the figures is inverted, elements described as "below" or "beneath" other elements or features would then be "above" or "over" the other elements or features. Thus, the exemplary term "below" can encompass both positions and orientations of above and below. A device may be otherwise oriented (rotated 90 degrees or at other orientations) and the spatially relative descriptors used herein interpreted accordingly.

Further modifications and alternative embodiments will be apparent to those of ordinary skill in the art in view of the disclosure herein. For example, the systems may include additional components that were omitted from the diagrams and description for clarity of operation. Accordingly, this description is to be construed as illustrative only and is for the purpose of teaching those skilled in the art the general manner of carrying out the systems and methods of the present disclosure. It is to be understood that the various embodiments shown and described herein are to be taken as exemplary. Elements and materials, and arrangements of those elements and materials, may be substituted for those illustrated and described herein, parts and processes may be reversed, and certain features of the present teachings may be utilized independently, all as would be apparent to one skilled in the art after having the benefit of the description herein. Changes may be made in the elements described herein without departing from the scope of the present invention as defined by the claims.

It is to be understood that the particular examples and embodiments set forth herein are non-limiting, and modifications to structure, dimensions, materials, and methodologies may be made without departing from the scope of the present disclosure. Other embodiments in accordance with the present disclosure will be apparent to those skilled in the art from consideration of the specification and practice of the invention disclosed herein. It is intended that the specification and examples be considered as exemplary only.

## Claims

1. A biopsy needle for penetrating soft tissue, comprising:
an elongate body (17) extending between a proximal end (34) and a distal end (36), the elongate body (17) having a central longitudinal axis (L); and
a tissue piercing tip (18) at the distal end (36) of the elongate body (17), the tissue piercing tip (18) comprising:
a first set of facets (25) defined by three first faces (35) and three first straight cutting edges (45), wherein each first straight cutting edge (45) is formed by adjacent faces of the three first faces (35), and wherein each first straight cutting edge (45) forms a first angle (θ₁) relative to the central longitudinal axis (L), and
a second set of facets (27) positioned distal to the first set of facets, the second set of facets (27) being defined by three second faces (37) and three second straight cutting edges (47), wherein each second straight cutting edge (47) is formed by adjacent faces of the three second faces (37), and wherein each second straight cutting edge (47) forms a second angle (θ₂) relative to the central longitudinal axis (L), the second angle (θ₂) being smaller than the first angle (θ₁), the three second straight cutting edges (47) converging to form a distal end point (19) of the biopsy needle.

2. The biopsy needle of claim 1, wherein the biopsy needle is a needle having dimensions corresponding to a seven-gauge (7-G) needle through a twelve-gauge (12-G) needle, preferably wherein the biopsy needle is a nine-gauge (9-G) needle or a seven-gauge (7-G) needle.

3. The biopsy needle of claim 1 or 2, wherein the first angle (θ₁) ranges between about 17 degrees and about 23 degrees relative to the central longitudinal axis.

4. The biopsy needle of any one of claims 1-3, wherein the second angle (θ₂) ranges between about 9 degrees and about 15 degrees relative to the central longitudinal axis.

5. The biopsy needle of any one of claims 1-4 wherein a diameter of the elongate body (17) is greater than a diameter of the tissue piercing tip (18).

6. The biopsy needle of any one of claims 1-5, wherein the elongate body (17) includes a sidewall (13) defining a lumen (24).

7. The biopsy needle of claim 6, wherein the elongate body (17) includes a tissue receiving aperture (20) in the sidewall (13), the tissue receiving aperture (20) being positioned adjacent to the tissue piercing tip (18) in the distal end (36) of the elongate body (17).

8. The biopsy needle of claim 7, wherein a distance between the distal end point (19) and the tissue receiving aperture (20) defines a dead space, the dead space ranging from about 8 mm to about 11 mm.

9. The biopsy needle of claim 8, wherein the first angle is about 20 degrees, the dead space is about 8.4 mm, and the second angle is about 9 degrees, or the second angle is about 12 degrees, or the second angle is about 15 degrees.

10. The biopsy needle of any one of claims 1-9, wherein the three first faces (35) are all symmetrical to each other and the three first straight cutting edges (45) are all symmetrical to each other.

11. The biopsy needle of any one of claims 1-10, wherein the three second faces (37) are all symmetrical to each other and the three second straight cutting edges (47) are all symmetrical to each other.

12. The biopsy needle of any one of claims 1-11, wherein the tissue piercing tip (18) comprises one or more reliefs (50).

13. The biopsy needle of claim 12, wherein the tissue piercing tip (18) comprises three reliefs (50) extending along the central longitudinal axis, each of the reliefs (50) extending along a respective facet surface (29) of the tissue piercing tip (18) that is formed by a respective pair of first (25) and second (27) facets.

14. The biopsy needle of claim 13, wherein each of the reliefs (50) forms a concave surface extending along the facet surface (29) between the cutting edges (49), the concave surface being defined by a radius of curvature and a lateral span.

15. The biopsy needle of claim 14, wherein the radius of curvature is between about 1.9 mm to about 3.7 mm and the lateral span is between about 6.4 mm and about 8.3 mm.

16. The biopsy needle of any preceding claim, wherein the three first faces (35) and the three second faces (37) are all planar surfaces.

17. A biopsy needle set for penetrating tissue, comprising:
the biopsy needle of any of claims 1, 3, 4, or 12 to 15 wherein the elongate body (17) comprises an outer cannula defining a central lumen extending along the longitudinal axis, the outer cannula including the tissue piercing tip at a distal end of the outer cannula and a tissue receiving aperture adjacent the tissue piercing tip; and
an inner cannula disposed within the central lumen of the outer cannula, the inner cannula being configured to slide relative to the outer cannula to close the tissue receiving aperture.

18. The biopsy needle set of claim 17, wherein the inner cannula includes an open distal end that is surrounded by an annular cutting blade.

19. The biopsy needle set of claim 18, wherein the outer cannula includes a cutting board disposed in the central lumen in a position distal to the tissue receiving aperture, the cutting board being configured to seal the open distal end of the inner cannula when the inner cannula is in contact with the cutting board.

20. A biopsy device, comprising:
a body portion; and
the biopsy needle set of any of claims 17 to 19 attached to the body portion, the body portion including a drive assembly configured to drive movement of the outer cannula and the inner cannula of the biopsy needle set.

21. The biopsy device of claim 20, wherein the biopsy needle set is disposable.

22. A biopsy needle set for penetrating soft tissue, comprising:
the biopsy needle of claim 1, wherein the elongate body (17) comprises an inner cannula extending between a proximal end and a distal end, the inner cannula including the tissue piercing tip at a distal end of the inner cannula and a tissue receiving aperture adjacent the tissue piercing tip, the three second cutting edges converging to form a distal end point of the inner cannula; and
an outer cannula defining a central lumen extending along a longitudinal axis, wherein the inner cannula is disposed within the central lumen of the outer cannula, the outer cannula being configured to slide relative to the inner cannula to close the tissue receiving aperture.

## Patentansprüche

1. Biopsienadel zum Durchdringen von Weichgewebe, umfassend:
einen langgestreckten Körper (17), der zwischen einem proximalen Ende (34) und einem distalen Ende (36) verläuft, wobei der langgestreckte Körper (17) eine zentrale Längsachse (L) aufweist; und
eine gewebestechende Spitze (18) an dem distalen Ende (36) des langgestreckten Körpers (17), wobei die gewebestechende Spitze (18) umfasst:
einen ersten Satz von Facetten (25), die durch drei erste Flächen (35) und drei erste gerade Schneidkanten (45) definiert sind, wobei jede erste gerade Schneidkante (45) durch benachbarte Flächen der drei ersten Flächen (35) gebildet ist und wobei jede erste gerade Schneidkante (45) einen ersten Winkel (θ₁) relativ zu der zentralen Längsachse (L) bildet, und
einen zweiten Satz von Facetten (27), der distal zu dem ersten Satz von Facetten angeordnet ist, wobei der zweite Satz von Facetten (27) durch drei zweite Flächen (37) und drei zweite gerade Schneidkanten (47) definiert ist, wobei jede zweite gerade Schneidkante (47) durch benachbarte Flächen der drei zweiten Flächen (37) gebildet ist, und wobei jede zweite gerade Schneidkante (47) einen zweiten Winkel (θ₂) relativ zu der zentralen Längsachse (L) bildet, wobei der zweite Winkel (θ₂) kleiner als der erste Winkel (θ₁) ist, wobei die drei zweiten geraden Schneidkanten (47) konvergieren, um einen distalen Endpunkt (19) der Biopsienadel zu bilden.

2. Biopsienadel nach Anspruch 1, wobei die Biopsienadel eine Nadel ist, die Abmessungen aufweist, die einer Sieben-Gauge(7-G)-Nadel bis einer Zwölf-Gauge(12-G)-Nadel entsprechen, wobei die Biopsienadel vorzugsweise eine Neun-Gauge(9-G)-Nadel oder eine Sieben-Gauge(7-G)-Nadel ist.

3. Biopsienadel nach Anspruch 1 oder 2, wobei der erste Winkel (θ₁) in dem Bereich zwischen etwa 17 Grad und etwa 23 Grad relativ zu der zentralen Längsachse liegt.

4. Biopsienadel nach einem der Ansprüche 1-3, wobei der zweite Winkel (θ₂) in dem Bereich zwischen etwa 9 Grad und etwa 15 Grad relativ zu der zentralen Längsachse liegt.

5. Biopsienadel nach einem der Ansprüche 1-4, wobei ein Durchmesser des langgestreckten Körpers (17) größer als ein Durchmesser der gewebestechenden Spitze (18) ist.

6. Biopsienadel nach einem der Ansprüche 1-5, wobei der langgestreckte Körper (17) eine Seitenwand (13) aufweist, die ein Lumen (24) definiert.

7. Biopsienadel nach Anspruch 6, wobei der langgestreckte Körper (17) eine Gewebeaufnahmeöffnung (20) in der Seitenwand (13) aufweist, wobei die Gewebeaufnahmeöffnung (20) benachbart zu der gewebestechenden Spitze (18) in dem distalen Ende (36) des langgestreckten Körpers (17) angeordnet ist.

8. Biopsienadel nach Anspruch 7, wobei ein Abstand zwischen dem distalen Endpunkt (19) und der Gewebeaufnahmeöffnung (20) einen Totraum definiert, wobei der Totraum in dem Bereich von etwa 8 mm bis etwa 11 mm liegt.

9. Biopsienadel nach Anspruch 8, wobei der erste Winkel etwa 20 Grad beträgt, der Totraum etwa 8,4 mm beträgt und der zweite Winkel etwa 9 Grad beträgt oder der zweite Winkel etwa 12 Grad beträgt oder der zweite Winkel etwa 15 Grad beträgt.

10. Biopsienadel nach einem der Ansprüche 1-9, wobei die drei ersten Flächen (35) alle symmetrisch zueinander sind und die drei ersten geraden Schneidkanten (45) alle symmetrisch zueinander sind.

11. Biopsienadel nach einem der Ansprüche 1-10, wobei die drei zweiten Flächen (37) alle symmetrisch zueinander sind und die drei zweiten geraden Schneidkanten (47) alle symmetrisch zueinander sind.

12. Biopsienadel nach einem der Ansprüche 1-11, wobei die gewebestechende Spitze (18) eine oder mehrere Aussparungen (50) umfasst.

13. Biopsienadel nach Anspruch 12, wobei die gewebestechende Spitze (18) drei Aussparungen (50) umfasst, die entlang der zentralen Längsachse verlaufen, wobei jede der Aussparungen (50) entlang einer jeweiligen Facettenoberfläche (29) der gewebestechenden Spitze (18) verläuft, die durch ein entsprechendes Paar von ersten (25) und zweiten (27) Facetten gebildet wird.

14. Biopsienadel nach Anspruch 13, wobei jede der Aussparungen (50) eine konkave Oberfläche bildet, die entlang der Facettenoberfläche (29) zwischen den Schneidkanten (49) verläuft, wobei die konkave Oberfläche durch einen Krümmungsradius und eine laterale Spannweite definiert ist.

15. Biopsienadel nach Anspruch 14, wobei der Krümmungsradius zwischen etwa 1,9 mm und etwa 3,7 mm beträgt und die laterale Spannweite zwischen etwa 6,4 mm und etwa 8,3 mm beträgt.

16. Biopsienadel nach einem der vorstehenden Ansprüche, wobei die drei ersten Flächen (35) und die drei zweiten Flächen (37) alle planare Oberflächen sind.

17. Biopsienadelsatz zum Durchdringen von Gewebe, umfassend:
die Biopsienadel nach einem der Ansprüche 1, 3, 4 oder 12 bis 15, wobei der langgestreckte Körper (17) eine äußere Kanüle umfasst, die ein zentrales Lumen definiert, das entlang der Längsachse verläuft, wobei die äußere Kanüle die gewebestechende Spitze an einem distalen Ende der äußeren Kanüle und eine Gewebeaufnahmeöffnung benachbart zu der gewebestechenden Spitze aufweist; und eine innere Kanüle, die innerhalb des zentralen Lumens der äußeren Kanüle angeordnet ist, wobei die innere Kanüle dazu ausgelegt ist, relativ zu der äußeren Kanüle zu gleiten, um die Gewebeaufnahmeöffnung zu verschließen.

18. Biopsienadelsatz nach Anspruch 17, wobei die innere Kanüle ein offenes distales Ende aufweist, das von einer ringförmigen Schneidklinge umgeben ist.

19. Biopsienadelsatz nach Anspruch 18, wobei die äußere Kanüle eine Schneidplatte aufweist, die in dem zentralen Lumen in einer Position distal zu der Gewebeaufnahmeöffnung angeordnet ist, wobei die Schneidplatte dazu ausgelegt ist, das offene distale Ende der inneren Kanüle abzudichten, wenn sich die innere Kanüle in Kontakt mit der Schneidplatte befindet.

20. Biopsievorrichtung umfassend:
einen Körperabschnitt; und
den Biopsienadelsatz nach einem der Ansprüche 17 bis 19,
der an dem Körperabschnitt angebracht ist, wobei der Körperabschnitt eine Antriebsanordnung umfasst, die dazu ausgelegt ist, Bewegung der äußeren Kanüle und der inneren Kanüle des Biopsienadelsatzes anzutreiben.

21. Biopsievorrichtung nach Anspruch 20, wobei der Biopsienadelsatz wegwerfbar ist.

22. Biopsienadelsatz zum Durchdringen von Weichgewebe, umfassend:
die Biopsienadel nach Anspruch 1, wobei der langgestreckte Körper (17) eine innere Kanüle umfasst,
die zwischen einem proximalen Ende und einem distalen Ende verläuft, wobei die innere Kanüle die gewebestechende Spitze an einem distalen Ende der inneren Kanüle und eine Gewebeaufnahmeöffnung benachbart zu der gewebestechenden Spitze aufweist, wobei die drei zweiten Schneidkanten konvergieren, um einen distalen Endpunkt der inneren Kanüle zu bilden; und
eine äußere Kanüle, die ein zentrales Lumen definiert,
das entlang einer Längsachse verläuft, wobei die innere Kanüle innerhalb des zentralen Lumens der äußeren Kanüle angeordnet ist, wobei die äußere Kanüle dazu ausgelegt ist, relativ zu der inneren Kanüle zu gleiten, um die Gewebeaufnahmeöffnung zu verschließen.

## Revendications

1. Aiguille de biopsie pour pénétrer les tissus mous, comprenant :
un corps allongé (17) s'étendant entre une extrémité proximale (34) et une extrémité distale (36), le corps allongé (17) ayant un axe longitudinal central (L) ; et
une pointe de perçage de tissu (18) à l'extrémité distale (36) du corps allongé (17), la pointe de perçage de tissu (18) comprenant :
un premier ensemble de facettes (25) définies par trois premières faces (35) et trois premiers bords de coupe rectilignes (45), chaque premier bord de coupe rectiligne (45) étant formé par des faces adjacentes des trois premières faces (35), et chaque premier bord de coupe rectiligne (45) formant un premier angle (θ₁) par rapport à l'axe longitudinal central (L), et
un second ensemble de facettes (27) positionné en position distale du premier ensemble de facettes, le second ensemble de facettes (27) étant défini par trois secondes faces (37) et trois seconds bords de coupe rectilignes (47), chaque second bord de coupe rectiligne (47) étant formé par des faces adjacentes des trois secondes faces (37), et chaque second bord de coupe rectiligne (47) formant un second angle (θ₂) par rapport à l'axe longitudinal central (L), le second angle (θ₂) étant inférieur au premier angle (θ₁), les trois seconds bords de coupe rectilignes (47) convergeant pour former un point d'extrémité distale (19) de l'aiguille de biopsie.

2. Aiguille de biopsie selon la revendication 1, l'aiguille de biopsie étant une aiguille ayant des dimensions correspondant à une aiguille de calibre sept (7-G) à une aiguille de calibre douze (12-G), de préférence l'aiguille de biopsie étant une aiguille de calibre neuf (9-G) ou une aiguille de calibre sept (7-G).

3. Aiguille de biopsie selon la revendication 1 ou 2, le premier angle (θ₁) étant compris entre environ 17 degrés et environ 23 degrés par rapport à l'axe longitudinal central.

4. Aiguille de biopsie selon l'une quelconque des revendications 1 à 3, le second angle (θ₂) étant compris entre environ 9 degrés et environ 15 degrés par rapport à l'axe longitudinal central.

5. Aiguille de biopsie selon l'une quelconque des revendications 1 à 4, un diamètre du corps allongé (17) étant supérieur au diamètre de la pointe de perçage de tissu (18).

6. Aiguille de biopsie selon l'une quelconque des revendications 1 à 5, le corps allongé (17) comprenant une paroi latérale (13) définissant une lumière (24).

7. Aiguille de biopsie selon la revendication 6, le corps allongé (17) comprenant une ouverture de réception de tissu (20) dans la paroi latérale (13), l'ouverture de réception de tissu (20) étant positionnée adjacente à la pointe de perçage de tissu (18) dans l'extrémité distale (36) du corps allongé (17).

8. Aiguille de biopsie selon la revendication 7, une distance entre le point d'extrémité distale (19) et l'ouverture de réception de tissu (20) définissant un espace mort, l'espace mort allant d'environ 8 mm à environ 11 mm.

9. Aiguille de biopsie selon la revendication 8, le premier angle étant d'environ 20 degrés, l'espace mort étant d'environ 8,4 mm, et le second angle étant d'environ 9 degrés, ou le second angle étant d'environ 12 degrés, ou le second angle étant d'environ 15 degrés.

10. Aiguille de biopsie selon l'une quelconque des revendications 1 à 9, les trois premières faces (35) étant toutes symétriques l'une par rapport à l'autre et les trois premiers bords de coupe rectilignes (45) étant tous symétriques l'un par rapport à l'autre.

11. Aiguille de biopsie selon l'une quelconque des revendications 1 à 10, les trois secondes faces (37) étant toutes symétriques l'une par rapport à l'autre et les trois seconds bords de coupe rectilignes (47) étant tous symétriques l'un par rapport à l'autre.

12. Aiguille de biopsie selon l'une quelconque des revendications 1 à 11, la pointe de perçage de tissu (18) comprenant un ou plusieurs reliefs (50).

13. Aiguille de biopsie selon la revendication 12, la pointe de perçage de tissu (18) comprenant trois reliefs (50) s'étendant le long de l'axe longitudinal central, chacun des reliefs (50) s'étendant le long d'une surface de facette respective (29) de la pointe de perçage de tissu (18) qui est formée par une paire respective de première (25) et seconde (27) facettes.

14. Aiguille de biopsie selon la revendication 13, chacun des reliefs (50) formant une surface concave s'étendant le long de la surface de facette (29) entre les bords de coupe (49), la surface concave étant définie par un rayon de courbure et une portée latérale.

15. Aiguille de biopsie selon la revendication 14, le rayon de courbure étant compris entre environ 1,9 mm et environ 3,7 mm et la portée latérale étant comprise entre environ 6,4 mm et environ 8,3 mm.

16. Aiguille de biopsie selon l'une quelconque des revendications précédentes, les trois premières faces (35) et les trois secondes faces (37) étant toutes des surfaces planes.

17. Ensemble d'aiguilles de biopsie pour pénétrer dans un tissu, comprenant :
l'aiguille de biopsie selon l'une quelconque des revendications 1, 3, 4 ou 12 à 15, le corps allongé (17) comprenant une canule extérieure définissant une lumière centrale s'étendant le long de l'axe longitudinal, la canule extérieure comprenant la pointe de perçage de tissu à une extrémité distale de la canule extérieure et
une ouverture de réception de tissu adjacente à la pointe de perçage de tissu ; et
une canule intérieure disposée à l'intérieur de la lumière centrale de la canule extérieure, la canule intérieure étant configurée pour coulisser par rapport à la canule extérieure afin de fermer l'ouverture de réception de tissu.

18. Ensemble d'aiguilles de biopsie selon la revendication 17, la canule intérieure comprenant une extrémité distale ouverte qui est entourée par une lame de coupe annulaire.

19. Ensemble d'aiguilles de biopsie selon la revendication 18, la canule extérieure comprenant une planche à découper disposée dans la lumière centrale dans une position distale par rapport à l'ouverture de réception de tissu, la planche à découper étant configurée pour sceller l'extrémité distale ouverte de la canule intérieure lorsque la canule intérieure est en contact avec la planche à découper.

20. Dispositif de biopsie, comprenant :
une partie de corps ; et
l'ensemble d'aiguilles de biopsie selon l'une quelconque des revendications 17 à 19, fixé à la partie de corps,
la partie de corps comprenant un ensemble d'entraînement configuré pour entraîner le mouvement de la canule extérieure et de la canule intérieure de l'ensemble d'aiguilles de biopsie.

21. Dispositif de biopsie selon la revendication 20, l'ensemble d'aiguilles de biopsie étant jetable.

22. Ensemble d'aiguilles de biopsie pour pénétrer dans les tissus mous, comprenant :
l'aiguille de biopsie selon la revendication 1, le corps allongé (17) comprenant une canule intérieure s'étendant entre une extrémité proximale et une extrémité distale,
la canule intérieure comprenant la pointe de perçage de tissu à une extrémité distale de la canule intérieure et
une ouverture de réception de tissu adjacente à la pointe de perçage de tissu, les trois seconds bords de coupe rectilignes convergeant pour former un point d'extrémité distale de la canule intérieure ; et
une canule extérieure définissant une lumière centrale s'étendant le long d'un axe longitudinal, la canule intérieure étant disposée à l'intérieur de la lumière centrale de la canule extérieure, la canule extérieure étant configurée pour coulisser par rapport à la canule intérieure afin de fermer l'ouverture de réception de tissu.
